# EUROPEAN PATENT SPECIFICATION

(11) **EP 1 962 712 B1**
(45) Date of publication and mention of the grant of the patent: **06.10.2010**
(21) Application number: 05850973.8
(22) Date of filing: 06.12.2005
(51) Int. Cl.: A61B 19/02, B65D 77/20

(54) **DISPOSABLE SAFETY "PATIENT KIT" FOR MEDICAL DEVICES**
EINWEG-SICHERHEITSPATIENTENKIT FÜR MEDIZINPRODUKTE
BOÎTIER DE SECURITE JETABLE POUR APPAREILS MEDICAUX POUR UN PATIENT

(43) Date of publication of application: 03.09.2008
(73) Proprietor: Sgarabottolo, Fabio, 35030 Cervarese S. Croce (IT)
(72) Inventor: Sgarabottolo, Fabio, 35030 Cervarese S. Croce (IT)
(74) Representative: Vinci, Marcello
(86) International application number: PCT/IT2005/000717
(87) International publication number: WO 2007/066359

(56) References cited:
- EP-A- 0 896 931
- US-A- 3 806 618
- US-A- 4 803 048
- US-A- 5 392 917
- US-B1- 6 286 705

## Description

The present patent relates to containers used in hospitals, analysis laboratories, testing points or treatment centres in general and in particular concerns a new sealed disposable safety patient kit with container for test tubes, vials or other medical devices.

The use of test tubes, vials or containers of other types in medical-hospital environments for the collection of body fluids and biological samples taken from the patient for the purposes of analysis is known.

The organic sample to be analysed is placed in said test tubes or vials when it is taken from the patient.

Subsequently, each of said test tubes or vials, designed to contain the sample to be analysed, is marked with one or more labels indicating, for example, the name and/or identification code of the patient and/or the type of analysis to be performed on the sample taken and/or the origin and/or any other information necessary for the laboratory and the person taking the sample.

The test tubes and/or vials are labelled before the sample is taken, so that the medical or nursing staff taking the sample already have the exact number of test tubes and/or vials to be used, each showing precise indications concerning the sample to be taken, and further labels necessary to identify other medical equipment or devices for sampling.

After the sample has been taken, said test tubes and/or vials, or other containers designed to contain the samples for analysis, are sent to the analysis laboratory.

If the purposely printed identification labels were applied manually to the test tube or vial after the sample has been taken, there would be the risk of confusing the test tubes or vials of different patients or different types, therefore causing serious errors, with possible consequences for the health of the patient and/or delays in performance of the analysis or the beginning of appropriate treatment, with the need to repeat the test.

On the other hand, it is not infrequent for the test tubes or vials marked manually prior to the test to be confused, with consequent errors and also delays in performance of the analysis and communication of the results. It is of fundamental importance for the health of the patients that for each of said patients tested, appropriate devices be used, correctly selected and labelled and with long-term batch traceability.

US 4 803 048, US 5 392 917, EP 0 896 931, US 6 286 705 disclose containers for medical devices or samples, each container comprising at least one cover with opening features, said cover having the function to protect taken samples or held surgical instruments. The two-part form of claim 1 is based on document US 4 803 048.

The subject of the present invention is a new pealed disposable safety patient kit with container for test tubes and/or vials or other medical devices, for use by hospitals, analysis laboratories, testing points or treatment centres in general.

The main aim of the present invention is to contain the test tubes and/or vials and/or other medical devices and/or other auxiliary labels of one single patient, distinguishing them and separating them from those of other patients.

A further aim of the present invention is to prevent confusion between test tubes and/or vials of different patients.

A further aim of the present invention is to prevent loss of the test tubes and/or vials and/or material tested.

A further aim of the present invention is to prevent deliberate malicious exchange or stealing or tampering with the test tubes and/or vials without leaving evidence of these operations.

These and other aims, direct and complementary, are achieved by the new sealed disposable safety patient kit with container for test tubes and/or vials or other medical devices, for use by hospitals or for treatment centres in general, with related adhesive closing and identification sheet.

The container is made preferably of plastic and comprises one or more apertures for insertion of the objects to be contained.

In the preferred embodiment of the invention, said container has a parallelepiped shape, with aperture obtained on its upper wall.

The test tubes and/or vials, in turn appropriately marked, and/or auxiliary labels and/or the other medical devices are placed inside said container. Said aperture of the container is then closed and sealed by means of a closing element or sheet made for example of paper, plastic-coated paper or plastic.

Said closing element can be for example an adhesive or partially adhesive sheet, i.e. provided with a layer of adhesive distributed at least on the surface in contact with said container.

For example, said element or sheet is adhesive only at the edges, where it comes into contact with the flat edges of said aperture of the container.

This prevents malicious or accidental outflow of the material contained in the container.

The name and/or the identification codes of the patient and/or the type and quantity of samples to be taken and/or the date on which the sample was taken and/or the test centre and/or any other information useful for the medical, nursing or technical staff are furthermore printed on said closing element.

The container, prepared as described above, is delivered to the patient or to the medical, technical or nursing staff, who use it in the hospital department or at the place where the sample is taken (domicile of the patient within or outside the facility) or in the test centres or analysis laboratories.

In this way, there is absolutely no risk of losing the test tubes and/or vials, or of prescribing inappropriate treatment for the patient as a result of incorrect diagnoses caused by errors in the pre-testing phase.

The procedure for depositing the test tubes and/or vials or other medical devices inside the container can be performed manually or, preferably, via the use of automatic devices.

For example, said container, moving on a sliding belt, can stop at a first station where the test tubes and/or vials and/or auxiliary labels and/or other medical devices necessary for taking the sample are deposited inside the container.

Subsequently, the container in question stops at a station in which said closing element, printed with all the above-mentioned information, is applied to the aperture of the container for closing and identification purposes.

Said closing element furthermore comprises a protruding tab and one or more incisions or perforations, so that said sealed container can be opened by tearing, for use of the material contained.

It is also possible to print on said tab the identification data of the patient or of the material contained in the container.

The new patient kit can also be used as a safety element after the sample has been taken, during subsequent transport to the analysis laboratories.

For said purpose, according to the invention a further cover is used, separate from or integral with the container and applied to the container, to seal it, after said element or closing and identification sheet has been tom open. Said cover is of the perimetric seal type, with perimetric teeth that snap into place along all or part of the edge of the container, and can be furthermore provided with a lateral tab with perforations on the upper part of said cover, for the opening of at least a part of the additional cover.

The subsequent removal of said additional cover causes the breakage of said teeth, so that the cover cannot be re-positioned or it is evident that it has been tampered with.

The attached drawings show a practical embodiment of the invention for illustrative non-limiting purposes.

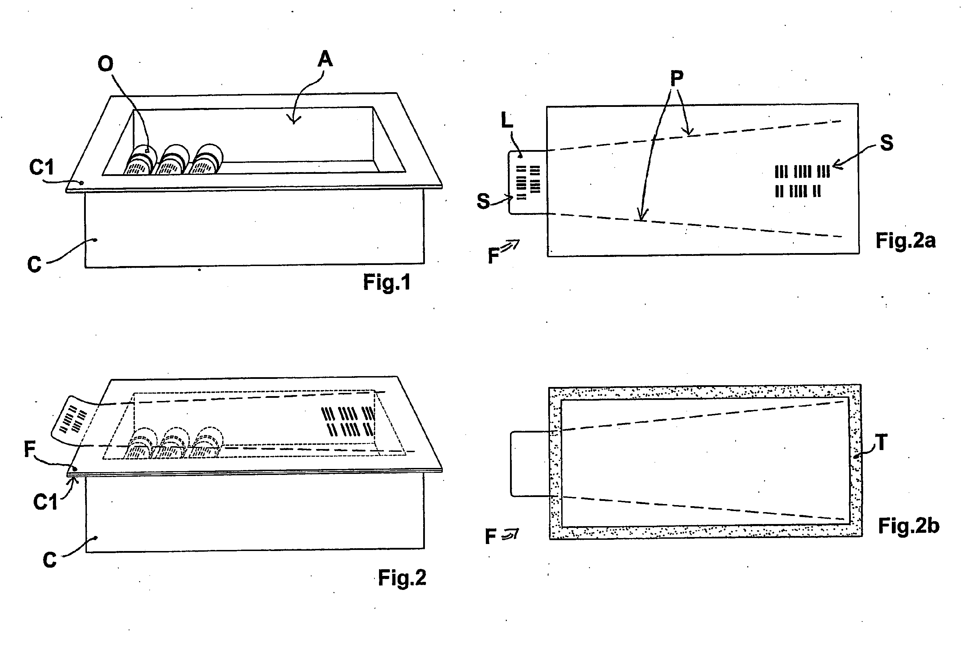

Figure 1 shows the container, while figure 2 shows the safety patient kit completely closed and sealed.

In figure 2a and 2b two views are shown, upper and lower, of the closing and identification element or sheet.

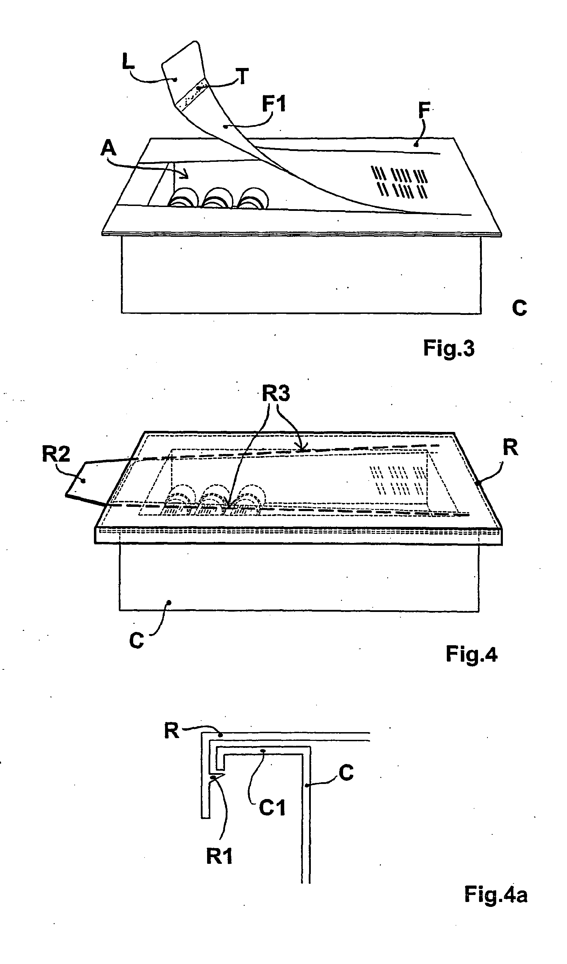

Figure 3 shows the patient kit partially open, while figure 4 shows the container closed with the additional cover.

Figure 4a schematises the closing method of the additional cover.

The new patient kit (K) comprises a container (C) having a parallelepiped, cubic or other shape and provided with at least one upper aperture (A) for the insertion of test tubes and/or vials (O) and/or auxiliary labels and/or other medical devices.

The patient kit (K) also comprises a closing and identification element (F), on which the identification data (S) of the patient and/or of the content and/or other information necessary for the person taking the sample are printed.

Said element (F) is an adhesive sheet wherein, for example, the adhesive layer (T) is distributed on the contact surface between said sheet (F) and the edge (C1) of said upper aperture (A) of the container (C).

Said closing element (F) comprises at least one tab (L), which can be provided with the identification data of the patient or of the content of the container, and one or more incisions or perforations (P) to permit opening of the sealed container.

In particular, said incisions or perforations (P) allow at least a part (F1) of said element (F) to be raised, giving access to the inside of the container (C).

After the opening of said closing element (F), it is possible to re-seal the container (C) via the use of an additional cover (R), detached from or integral with said container (C).

Said additional cover (R) is of the perimetric seal type, i.e. it comprises a plurality of teeth (R1) distributed along its perimeter, which snap into all or part of the edge (C1) of the container, preferably at the corners.

It can also have a lateral tab (R2) and perforations (R3) on the upper part, for tear-opening of at least part of the additional cover (R).

The re-opening of said additional cover (R) causes breakage of said teeth (R1).

Therefore, with reference to the preceding description and the attached drawings, the following claims are expressed.

## Claims

1. Safety patient kit (K) for test tubes and/or vials (O) and/or other medical devices for use in hospitals, private laboratories, test points or treatment centres in general, comprising a container (C) with at least one aperture (A) for the insertion of said test tubes and/or vials (O) and/or medical devices in general, and at least one element (F) for closing and sealing said aperture (A), comprising one or more incisions or perforations (P) and/or other systems for tear-opening of at least a part (F1) of said element (F), for access inside said container (C), **characterised in that** said element (F) is provided with identification data (S), in plain text or in code, relating to the patient and/or the content and/or the use of the content and/or the origin and/or any other information necessary to the person taking the sample, said element (F) being printed in the phase prior or subsequent to its application to the container (C), and wherein said kit (K) also comprises a further cover (R), detached from or integral with said container (C), said cover (R) being provided with:
• a plurality of teeth (R) distributed along all or part of the perimeter of said cover (R) and designed to snap in along all or part of the edge (C1) of said container (C), and wherein the subsequent removal of said cover (R) causes the breakage of said teeth (R1);
• a lateral tab (R2) and perforations (R3) for tear-opening of at least a part of said additional cover (R).

2. Safety patient kit (K) according to claim 1, **characterised in that** said closing and identification element (F) is an adhesive sheet comprising at least one protruding tab (L).

3. Safety patient kit (K) according to claims 1, 2, **characterised in that** said identification data are provided on any part of said element (F).

4. Safety patient kit (K) according to the preceding claims, **characterised in that** it is disposable.

5. Safety patient kit (K) according to the preceding claims, **characterised in that** it is positioned on a sliding belt and/or in another automatic device for the insertion of said test tubes and/or vials (O) and/or auxiliary labels and/or medical devices and/or for the application of said adhesive closing and identification sheet (F).

## Patentansprüche

1. Patienten-Sicherheitskit (K) für Reagenzgläser und/oder Ampullen (O) und/oder andere Medizinprodukte zum Gebrauch in Krankenhäusern, Privatlabors, Blutabnahmestellen oder Behandlungszentren im Allgemeinen, einen Behälter (C) umfassend mit wenigstens einer Öffnung (A) zum Einsetzen der besagten Reagenzgläser und/oder Ampullen (O) und/oder anderen Medizinprodukten im Allgemeinen, sowie wenigstens ein Element (F) zum Verschließen und Versiegeln besagter Öffnung (A), eine oder mehrere Einritzungen oder Perforationen (P) umfassend und/oder andere Systeme zum Aufreißen von wenigstens einem Teil (F1) des besagten Elements (F), um Zugang zum Inneren des Behälters (C) zu erlangen, **dadurch gekennzeichnet, dass** das besagte Element (F) Identifikationsdaten (S) in Volltext oder codierter Form über den Patienten und/oder den Inhalt und/oder den Gebrauch des Inhalts und/oder die Herkunft und/oder jegliche andere, für die die Probe nehmende Person erforderliche Information enthält, wobei besagtes Element (F) in der Phase vor oder nach seiner Anbringung am Behälter (C) bedruckt wird, und wobei besagter Kit (K) außerdem einen weiteren, separaten oder mit besagtem Behälter (C) eine Einheit bildenden Deckel (R) aufweist, wobei dieser Deckel (R) Folgendes umfasst:
• eine Vielzahl von über den gesamten Umfang des Deckels oder einen Teil desselben verteilter Zähne (R), die geeignet sind, über die gesamte Kante (C1) des besagten Behälters (C) oder einen Teil derselben einzurasten, wobei die nachfolgende Entfernung des besagten Deckels (R) den Bruch der besagten Zähne (R1) verursacht;
• eine Seitenlasche (R2) und Perforationen (R3) zum Aufreißen von wenigstens einem Teil des besagten zusätzlichen Deckels (R).

2. Patienten-Sicherheitskit (K) gemäß Patentanspruch 1, **dadurch gekennzeichnet, dass** das besagte Schließ- und Identifikationselement (F) ein Klebeblatt mit wenigsten einer herausragenden Lasche (L) ist.

3. Patienten-Sicherheitskit (K) gemäß Patentansprüchen 1, 2, **dadurch gekennzeichnet, dass** die besagten Identifikationsdaten auf einem jeglichen Teil des besagten Elements (F) aufgeführt sind.

4. Patienten-Sicherheitskit (K) gemäß vorstehenden Patentansprüchen, **dadurch gekennzeichnet, dass** es für den einmaligen Gebrauch bestimmt ist.

5. Patienten-Sicherheitskit (K) gemäß vorstehenden Patentansprüchen, **dadurch gekennzeichnet, dass** es auf einem laufenden Band und/oder in einer anderen, automatischen Vorrichtung zur Einfügung der besagten Reagenzgläser und/oder Ampullen (O) und/oder zusätzlicher Etiketten und/oder Medizinprodukte und/oder zur Anbringung der klebenden Schließ- und Identifikationsblätter (F) positioniert ist.

## Revendications

1. Trousse de sécurité pour patient (K) pour éprouvettes et/ou ampoules (O) et/ou autres dispositifs médicaux pour l'emploi en hôpitaux, laboratoires privés, lieux de prélèvement ou centres de traitement en général, comprenant un containeur (C) avec au moins une ouverture (A) pour l'introduction desdites éprouvettes et/ou ampoules (O) et/ou dispositifs médicaux en général, et au moins un élément (F) pour la fermeture et le cachetage de ladite ouverture (A), comprenant une ou plusieurs incisions ou perforations (P) et/ou autres systèmes pour l'ouverture par déchirure d'au moins une partie (F1) dudit élément (F), pour l'accès à l'intérieur dudit containeur (C), **caractérisée en ce que** ledit élément (F) est doté de données d'identification (S), avec texte en clair ou en code, concernant le patient et/ou le contenu et/ou l'emploi du contenu et/ou l'origine et/ou toute autre information nécessaire à la personne qui prélève l'échantillon, ledit élément (F) étant imprimé dans la phase avant ou successive à son application sur le containeur (C), et où ladite trousse (K) comprend également un couvercle supplémentaire (R), détaché ou solidaire dudit containeur (C), ledit couvercle (R) étant muni de :
• une pluralité de dents (R) distribuées le long de tout ou une partie du périmètre dudit couvercle (R) et conçues pour s'insérer par déclic le long de tout ou une partie du bord (C1) dudit containeur (C), et où la dépose successive dudit couvercle (R) cause la rupture desdites dents (R1);
• une languette latérale (R2) et des perforations (R3) pour l'ouverture par déchirure d'au moins une partie dudit couvercle supplémentaire (R).

2. Trousse de sécurité pour patient (K) selon la revendication 1, **caractérisée en ce que** ledit élément de fermeture et d'identification (F) est une feuille adhésive comprenant au moins une languette saillante (L).

3. Trousse de sécueité pour patient (K) selon les revendications 1 et 2, **caractérisée en ce que** lesdites données d'identification sont prévues sur toute partie dudit élément (F).

4. Trousse de sécurité pour patient (K) selon les revendications précédentes, **caractérisée en ce qu'**elle est jetable.

5. Trousse de sécurité pour patient (K) selon les revendications précédentes, **caractérisée en ce qu'**elle est positionnée sur une bande coulissante et/ou sur un autre dispositif automatique pour l'introduction desdites éprouvettes et/ou ampoules (O) et/ou étiquettes auxiliaires et/ou dispositifs médicaux et/ou pour l'application de ladite feuille adhésive de fermeture et d'identification (F).
